# EUROPEAN PATENT SPECIFICATION

(11) **EP 1 420 720 B1**
(45) Date of publication and mention of the grant of the patent: **19.09.2007**
(21) Application number: 02752685.4
(22) Date of filing: 06.08.2002
(51) Int. Cl.: A61F 2/86

(54) **STENT AND APPLICATOR**
STENT UND APPLIKATOR
STENT ET APPLICATEUR

(30) Priority: 23.08.2001 US 935487
(43) Date of publication of application: 26.05.2004
(73) Proprietor: Boston Scientific Limited, St. Michael, Barbados, West Indies (BB)
(72) Inventor: RIOUX, Robert, F., Ashland, MA 01721 (US); O'KEEFE, Christopher, R., Holliston, MA 01746 (US)
(74) Representative: Chapman, Paul William
(86) International application number: PCT/US2002/024707
(87) International publication number: WO 2003/017871

(56) References cited:
- US-A- 4 813 925
- US-A- 5 282 860
- US-A- 5 514 176
- US-A- 5 824 041

## Description

### Technical Field

This invention generally relates to stents for maintaining a body passageway open.

### Background Information

Bladder outlet obstruction is a common urological disorder that some men, especially men over fifty years of age, experience. Due to the anatomy of a male urinary system, one of the most common causes of bladder outlet obstruction is constriction of the urethra by a swollen or enlarged prostate. To alleviate the constriction created by the enlarged prostate, a medical professional may insert a catheter or a stent into the patient's urethra to reinforce a portion the urethra that the prostate surrounds (commonly refer to as the prostatic urethra) and to maintain an open passageway therethrough.

Generally, the catheters that are used to relieve urine retention are flexible tubular devices that are sized to extend from the patient's bladder through the entire urethra and terminate outside of the patient's body. These catheters, while effective at providing the patients with an open passageway for constant fluid drainage from the bladder, may cause patient discomfort because the catheters prevent the normal operation of the patients'external sphincters.

Some prostatic stents, because of their size and design, extend from the bladder through the prostatic urethra and terminate just prior to the patient's external sphincter. These stents, because of their position within the prostatic urethra, allow the external sphincter to function normally and thus preserve the patient's voluntary control over urination. U.S. Patent No. 5,514,176 describes a stent for placement in a body lumen. The stent is configured as a coil with adjacent loops that abut one another to yield a resilient configuration of generally fixed dimension. To remove the stent from the body lumen, and end of the stent is pulled so that adjacent loops separate, optimally completely from one another, into a pulled shape that is fully uncoiled.

### Summary of the Invention

The invention generally relates to draining fluid from the bladder of a male patient with a stent. Devices according to the invention are typically used in the treatment for male patients suffering from bladder outlet obstruction. It is an object of the invention to provide a stent to maintain an open passageway through the patient's prostatic urethra while also allowing normal operation of the patient's external sphincter such that the patient has control over the retention and discharge of fluids from the bladder. It is another object of the invention to provide a stent that is resistant to ingrowth of tissue and is also resistant to migration once positioned within the patient's urinary system.

It is noted initially that the directional terms proximal and distal require a point of reference. As used herein, the point of reference is from the perspective of the body of the patient. Therefore, the term proximal refers to a direction that points into the body of the patient, whereas the term distal refers to a direction that points out of the patient's body.

In general, in one aspect, the invention features a stent for use within a body lumen of a patient. The stent includes a coil segment encapsulated within a flexible polymer material. The coil segment of the stent has a distal portion, a middle portion, and a proximal portion. Each of the proximal and distal portions includes a diameter that is greater than the diameter of the middle portion to enhance retention and to inhibit movement of the stent after it is positioned and leftwithin the prostatic urethra. The coil segment defines a lumen extending therethrough and includes a wound element having one or more windings. The windings are spaced from each other along at least a portion of the length of the coil segment and may be reduced in width at least to an extent needed to pass the stent into the body lumen of the patient by winding of the wound element. The flexible polymer material that encapsulates the coil segment is disposed between the windings of the wound element. The flexible polymer material forms an imperforate flexible webbing between the windings that inhibits ingrowth of body tissues within the coil segment when the stent is placed within the body lumen of the patient so as to maintain the lumen of the coil segment open.

Embodiments of this aspect of the invention may include the following features. The wound element may be a wire of a biocompatible material, such as, for example, stainless steel, titanium, a nickel-titanium alloy, or a polymer. The cross-sectional shape of the wire influences the radial strength and flexibility of the coil segment. To allow for a sufficient combination of radial strength and flexibility, the cross-sectional area of the wire used to form the coil segment is in the range of from about 7.9 x 10⁻³ millimeters² to about 7.1 millimeters². For a wire with a circular cross-sectional shape, the cross-sectional area range given above corresponds to a diameter range of about 0.1 to about 3 millimeters.

In some embodiments, the windings may be separated at a distance from each other in the range of from about 0.5 millimeters to about 10 millimeters. Stents that include windings separated at a larger distance have greater flexibility than those stents with windings separated at a smaller distance. The flexible polymer material forms the imperforate flexible webbing adjoining each adjacent winding. An example of a flexible polymer material that is suitable for forming the imperforate flexible webbing is a low durometer silicone, specifically a silicone that has a Shore A hardness in the range of from about 0 to 60 durometers.

In general, in another aspect, the invention relates to a method of attaching the above-described stent to a delivery system. The delivery system includes a first element and a second element. At least one of the first and second elements is able to rotate. The first element has an outer diameter that is smaller than the diameter of the middle portion of the stent so as to be insertable within the lumen of the coil segment. The first element includes a first end and a second end. A connection member extends off of the first end to permit connection between the first element and the proximal end of the stent. The second element also has a first end and a second end. A connection member extends off of the first end of the second element and connects the distal portion of the stent to the delivery system. The method of attaching the stent to the delivery system includes providing both the stent and the delivery system, placing the first element of the delivery system within the lumen of the coil segment, attaching the connection member of the first element to the proximal portion of the stent, attaching the connection member of the second element to the distal portion of the stent, and rotating at least one of the first and the second elements to further wind the wound element to reduce the width of the stent at least to an extent needed to pass the stent into the urethra of the patient.

Embodiments of this aspect of the invention may include the following features. The second element of the delivery system may define a lumen extending therethrough. The lumen of the second element is sized so that the lumen may receive the first element of the delivery system. In some embodiments, the connection members located at the first ends of the first and second elements of the delivery system may include an opening sized to receive a hook extending from the proximal or distal end of the stent.

The stent can be positioned within a patient. To insert and position the stent, a medical professional, such as, for example, a physician, uses the delivery system as described above. A manufacturer may provide the physician with the stent attached to the delivery system. In this embodiment, the stent attached to the delivery system is wound onto at least a portion of the first element of the delivery system. The stent is wound at least to an extent needed to pass the stent into the urethra of the patient. To position the stent within the body of the patient, the medical professional first inserts the delivery system with the attached and wound stent into the urethra of the patient. Then, the medical professional advances the stent through the patient's urethra and positions the stent within the prostatic urethra. When properly positioned, the stent is located within the prostatic urethra of the patient and extends from the bladder opening to the proximal side of the external sphincter. After positioning the stent, the medical professional rotates at least one of the first and the second elements of the delivery system to at least partially unwind the stent, thereby expanding the width of the stent. Once the width of the stent has expanded the medical professional releases the stent from the connection members of the delivery system. After disconnecting the stent from the delivery system, the medical professional removes the delivery system from the patient's urethra and the stent remains in position within the patient's prostatic urethra maintaining an open passageway therethrough.

The foregoing and other objects, aspects, features, and advantages of the invention will become more apparent from the following description and from the claims.

### Brief Description of the Drawings

In the drawings, like reference characters generally refer to the same parts throughout the different views. Also, the drawings are not necessarily to scale, emphasis instead generally being placed upon illustrating the principles of the invention.

FIG. 1 is a schematic view of a male urinary system.

FIG. 2A is a front view of one embodiment of a prostatic stent of the invention.

FIG. 2B is a bottom view of one embodiment of a coil segment of the invention.

FIG. 3A is a cross-sectional view of the prostatic stent taken along a line AA in FIG. 2A.

FIG. 3B is another cross-sectional view of the prostatic stent taken along a line BB in FIG. 2A.

FIG. 4A is a cross-sectional view of the prostatic stent taken along a line CC in FIG. 2A.

FIG. 4B is another cross-sectional view of the prostatic stent of FIG. 2A after compression of the prostatic stent.

FIG. 4C is another cross-sectional view of the prostatic stent of FIG. 2A after bending of the prostatic stent has occurred.

FIG. 5A is a front view of the prostatic stent of FIG. 2A prior to further winding of a coil element to decrease an overall diameter of the prostatic stent.

FIG. 5B is a front view of the prostatic stent of FIG. 2A after winding of the coil element to decrease the overall diameter of the stent.

FIG. 6A is a front view of one embodiment of a prostatic stent.

FIG. 6B is a front view of another embodiment of a prostatic stent.

FIG. 7 is a front view of one embodiment of a delivery system.

FIG. 8 is a schematic view of the prostatic stent of FIG. 2A attached to the delivery system.

FIG. 9 is a schematic view of the prostatic stent with a decreased diameter attached to the delivery system.

FIG. 10 is a schematic view of the prostatic stent with decreased diameter and attached to the delivery system being inserted into a patient's urinary system.

FIG. 11 is an enlarged schematic view of the prostatic stent properly positioned within the patient's urinary system.

### Description

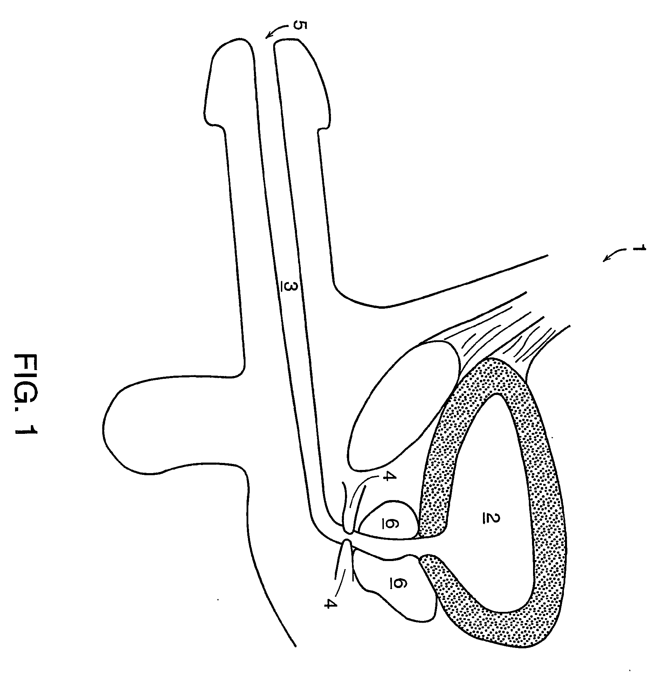

Urine retention and reduced urination are two common symptoms that some male patient afflicted with benign prostatic hyperplasia (BPH) endure. BPH is a medical condition in which a patient's prostate enlarges due to disease or decreasing hormone production. FIG. 1 shows a male urinary system 1, which includes a bladder 2, a urethra 3, an external sphincter 4, a meatus 5, and a prostate 6. The prostate 6 is a male reproductive organ that surrounds a section of the urethra 3 generally known as the prostatic urethra. Due to the prostate's 6 location, the male urinary system 1 may be constricted and thus obstructed when the patient's prostate 6 enlarges. One of the objects of the present invention is to maintain an open passageway from the patient's bladder 2 through the urethra 3 while simultaneously preserving the patient's voluntary control over urination by allowing the external sphincter 4 to open and close under patient control.

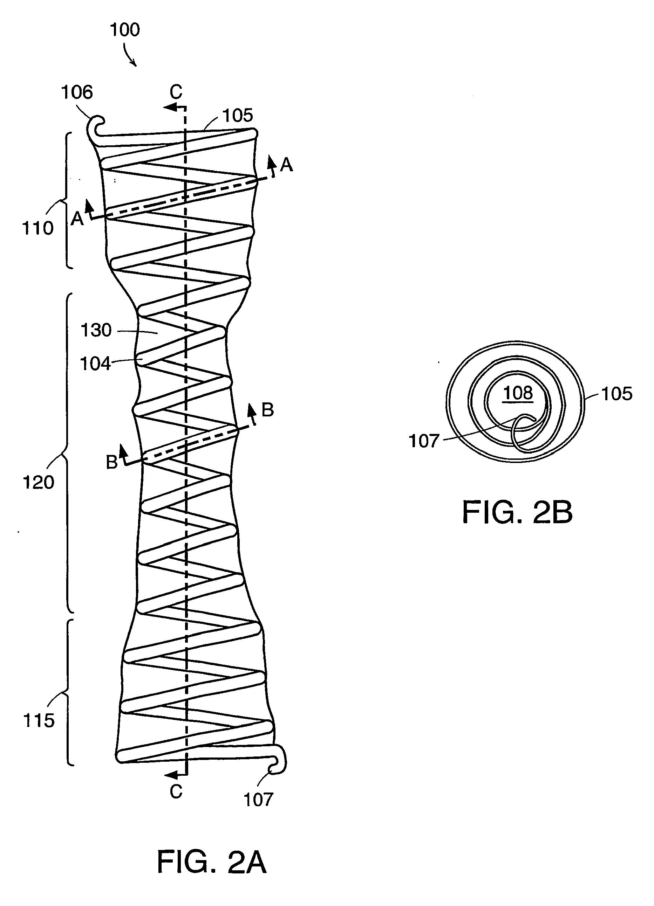

Referring to FIGS. 2A and 2B, a prostatic stent 100 comprises a coil segment 105 and an imperforate flexible webbing 130. Both the coil segment 105 and the imperforate flexible webbing 130 define a lumen 108 extending within the prostatic stent 100 that allows fluids, such as, urine to pass therethrough from the bladder of a patient. The coil segment 105 has one or more windings 104 spaced from each other along the length of the coil segment 105. The coil segment 105 may be made from an element that can be wound, such as, for example, a stainless steel wire. Alternatively, a wire of any biocompatible material, such as, for example, a polymer, titanium, or a nickel-titanium alloy may be used as well.

The wire used to form the coil segment 105 may have one of a variety of cross-sectional shapes, such as, for example, circular, square, rectangular, triangular, or trapezoidal. In the disclosed embodiment, the wire has a circular cross-sectional shape having a cross-sectional area defined by the wire's diameter. The diameter or thickness, or more generally the cross-sectional area or shape, of the wire selected for the coil segment 105, influences the radial strength as well as the flexibility of the prostatic stent 100. Thus, for the disclosed embodiment, the diameter of the wire selected needs to be sufficiently large to assure proper radial strength of the prostatic stent 100 is achieved to prevent against obstruction of the patient's prostatic urethra resulting from the constriction created by the patient's enlarged prostate. At the same time, however, the diameter of the wire also needs to be sufficiently thin to promote winding ease and flexibility of the prostatic stent 100 to accommodate the patient's anatomy. In one embodiment, the diameter of the round wire used to form the coil segment 105 is generally in the range of from about 0.1 millimeters to about 3 millimeters, which corresponds to a cross-sectional area in the range of about 7.9 x10-3 millimeters2 to about 7.1 millimeters2. In the disclosed embodiment, the wire's diameter is 1 millimeter, which corresponds to a cross-sectional area of about 0.20 millimeters2.

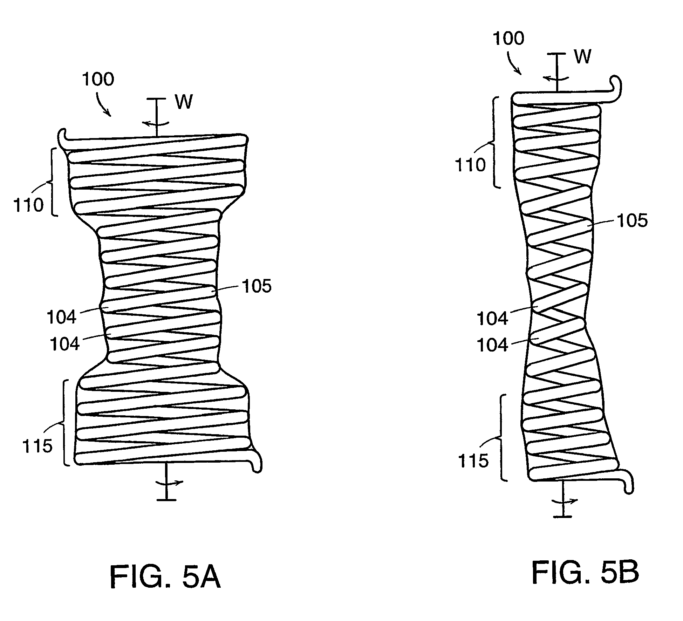

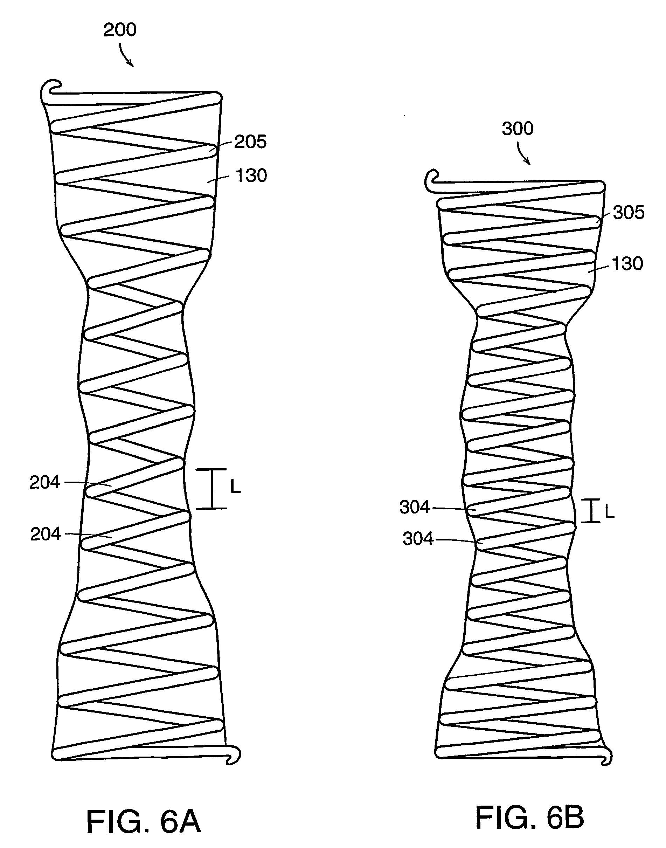

The wire is wound to form the coil segment 105 that includes a proximal portion 110, a middle portion 120, and a distal portion 115. Each of the proximal and distal portions 110, 115 may include a hook 106,107. The hooks 106,107 are positioned such that the coil segment 105 may be connected to a device used to wind the prostatic stent 100 to a smaller width and to deliver the prostatic stent 100 into the body of the patient. In the embodiment shown in FIG. 2A, the hooks 106,107 are positioned such that they extend lengthwise beyond the proximal and distal portions 110,115, respectively. The hooks 106,107 need not be positioned as shown in FIG. 2A to allow the coil segment 105 to connect to the device. Other hook positions are possible. For example, the hooks may be positioned such that they extend inward into the lumen 108 of the stent 100, as shown in FIG. 2B. The hooks shown in other drawings, such as FIGS. 5A, 5B, 6A, and 6B, also could be configured or positioned in other ways including as shown in FIG. 2B.

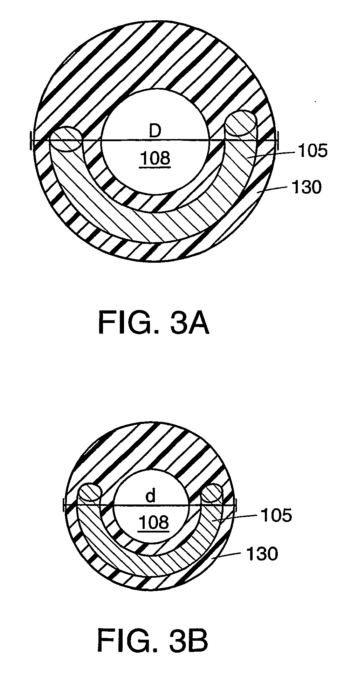

To retain proper positioning of the prostatic stent 100 when the stent is left within the patient's body and to inhibit movement of the positioned prostatic stent 100, each of the proximal and distal portions 110,115 have a greater diameter than the middle portion 120. In one disclosed embodiment, the diameter of the proximal and distal portions 110,115 is about 13.3mm [40 French], whereas the diameter of the middle portion 120 is about 8mm [24 French]. FIGS. 3A and 3B are cross-sectional views of the prostatic stent 100 showing the greater diameter, D, of the proximal portion 110 and a smaller diameter, d, of the middle portion 120, respectively. FIG. 3A shows a cross-section taken along a line AA in FIG. 2A (within the proximal portion 110). FIG. 3B shows a cross-section taken along a line BB in FIG. 2A (within the middle portion 120). When properly positioned within the patient's body, the prostatic stent 100 is located substantially within the patient's prostatic urethra with the proximal portion 110 located within the bladder opening and the distal portion 115 located on the proximal side of the patient's external sphincter 4, so as not to interfere with the normal operation of the external sphincter 4. The greater diameter, D, of the proximal and distal portions 110, 115 is sized such that the proximal and distal portions 110, 115 are in contact with and exert a compressive force on the patient's urethral wall, thereby anchoring the prostatic stent 100 within the patient's urinary system 1 and preventing the migration of the prostatic stent 100. The greater diameter of the proximal portion 110 prevents the distal migration of the prostatic stent 100 (down and out of the bladder opening). The greater diameter of the distal portion 115 prevents the proximal migration of the prostatic stent 100 (up into the bladder 2 of the patient).

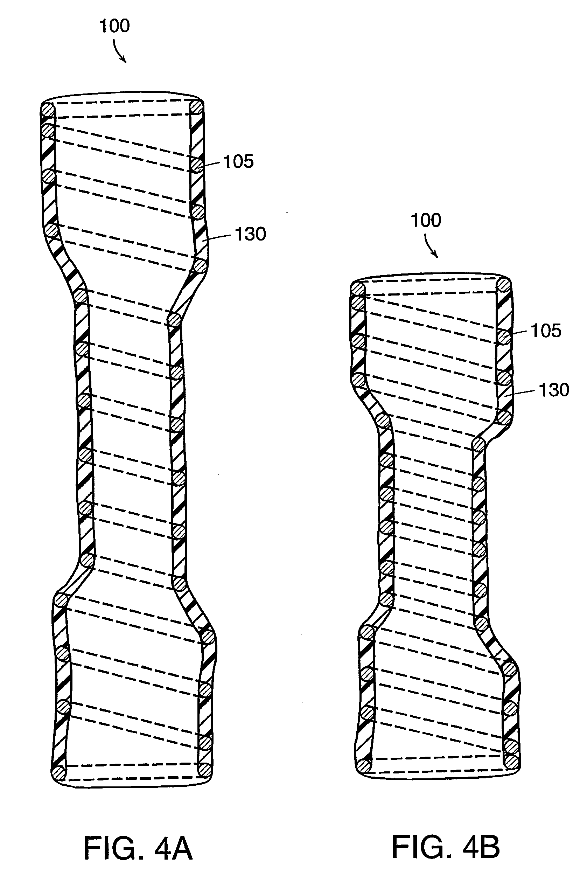

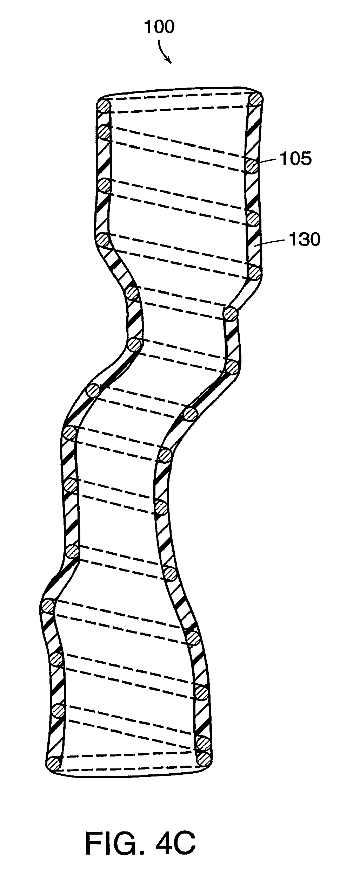

Referring to FIG. 4A, the imperforate flexible webbing 130 encapsulates the coil segment 105 and is disposed between adjacent windings 104. The imperforate flexible webbing 130 is a non-porous membrane that inhibits ingrowth of body tissue between the windings 104 of the coil segment 105 and prevents encrustation of the prostatic stent 100. In the disclosed embodiment, the imperforate flexible webbing 105 is made from silicone having a hardness of about 10 on the Shore A durometer scale. In other embodiments, the imperforate flexible webbing may be made from silicone having a hardness in the general range of about 0 to 60 on the Shore A durometer scale. Alternatively, the imperforate flexible webbing 130 may be made from any flexible biocompatible material that can stretch when the coil segment 105 is extended lengthwise, can collapse when the coil segment 105 is compressed lengthwise, and can flex when the coil segment is bent or deformed. FIG. 4B shows a similar cross-sectional view of the prostatic stent 100 as shown in FIG. 4A, but the coil segment 105 has been compressed lengthwise. To accommodate the compression of the coil segment 105, the imperforate flexible webbing 130 collapses between each winding 104. The imperforate flexible webbing 130 can also accommodate bending of the coil segment 105. As shown in cross-section in FIG. 4C, the imperforate flexible webbing 130 can flex in the same direction as the coil segment 105 without tearing.

Generally, the prostatic stent 100 is produced in three stages. First, an outer layer of the flexible imperforate webbing 130 is created by dipping an open-ended preform having a lumen extending within and a shape substantially equivalent to the shape of the coil segment 105 into a molten bath of silicone or other biocompatible polymer. The coil segment 105 is then placed within the preform in contact with the outer layer of the imperforate flexible webbing 130. Finally, the preform including the outer layer and the coil segment 105 is dipped again into the molten bath to form an inner layer of the imperforate flexible webbing 130 that adheres to the outer layer and encapsulates the coil segment 105. The fully formed prostatic stent may then be removed from the lumen of the preform. Alternatively, the prostatic stent 100 may be manufactured by dipping a cylindrical or hourglass shaped mandrel into the molten bath of silicone to create the inner layer of the imperforate flexible webbing 130 first. After the inner layer is formed, the coil segment 105 is secured to the inner layer, and then the mandrel with the inner layer and coil segment 105 attached is dipped again into the bath of silicone to form the outer layer of the imperforate flexible webbing 130. The outer layer of the imperforate flexible webbing 130, in combination with the inner layer, encapsulates the coil segment 105. Once the outer and inner layers of the imperforate flexible webbing 130 have solidified to form the imperforate flexible webbing130, the prostatic stent 100 may be removed from the mandrel. Other manufacturing methods may be used as well to encapsulate the coil segment 105 in the imperforate flexible webbing 130. For example, a manufacturer can dip the coil segment 105 directly into the molten bath of silicone. Generally, the manufacturer holds the coil segment 105 in an upright position as shown in FIG. 2A while dipping the coil segment 105 into the molten bath. To ensure that the imperforate flexible webbing is evenly distributed along the length of the coil segment 105, the manufacturer rotates the coil segment by 180 degrees about an axis that is perpendicular to the length of the coil segment 105.

The imperforate flexible webbing 130 links the windings 104 together and provides a barrier preventing the proliferation of connective tissue between and around the windings 104 when the prostatic stent 100 is positioned within the body of a patient. Growth of tissue between and around the windings 104 may lead to incorporation of a stent into the patient's urethral wall or occlusion and therefore obstruction of the stent. It will thus be appreciated that the imperforate flexible webbing 130 of the disclosed prostatic stent 100 prevents ingrowth of tissue and thereby prevents incorporation of the prostatic stent 100 within the urethra due to encrustation and obstruction due to epithelialization.

Before the prostatic stent 100 is inserted into the patient's urinary system, the width of the prostatic stent 100 is reduced to allow the prostatic stent 100 to easily pass through the patient's urethra. One may reduce the width of the prostatic stent 100 to have a largest outer diameter that is in a range between about 5.3mm [16 French] to about 6mm [18 French]. The width of the prostatic stent 100 may be temporarily reduced by twisting or winding the proximal portion 110 of the coil segment 105 about a longitudinal axis, W, while restraining movement of the distal portion 115, as shown in FIGS. 5A and 5B. Alternatively, twisting the distal portion 115 about the longitudinal axis, W, while restraining the proximal portion 110 may also reduce the width of the prostatic stent 100. The imperforate flexible webbing 130 is sufficiently pliable to twist along with the coil segment 105 without ripping or tearing.

One of the advantageous features of the prostatic stent 100 is its combination of radial strength and flexibility. The coil segment 105 with spaced windings 104 provides radial strength to the prostatic stent 100, while permitting the degree of flexibility necessary to conform to the patient's anatomy. In one embodiment the windings 104 are spaced such that a distance of about 1 millimeter exists between each adjacent winding. In other embodiments, where a greater amount of flexibility is desired, the windings 104 may be spaced at a greater distance, such as, for example, up to about 10 millimeters. FIG. 6A is an enlarged front view of one embodiment of a prostatic stent 200 having windings 204 of a coil segment 205 spaced at a large distance, L. Alternatively, if a greater amount of strength is required to maintain an open passageway through the patient's prostatic urethra, a medical professional may insert a prostatic stent 300 having windings 304 of a coil segment 305 closely spaced, at a smaller distance,1, as shown in FIG. 6B. To maintain a desirable amount of flexibility to be able to conform to the patient's anatomy, the windings 304 may be spaced at a distance no less than about 0.5 millimeters.

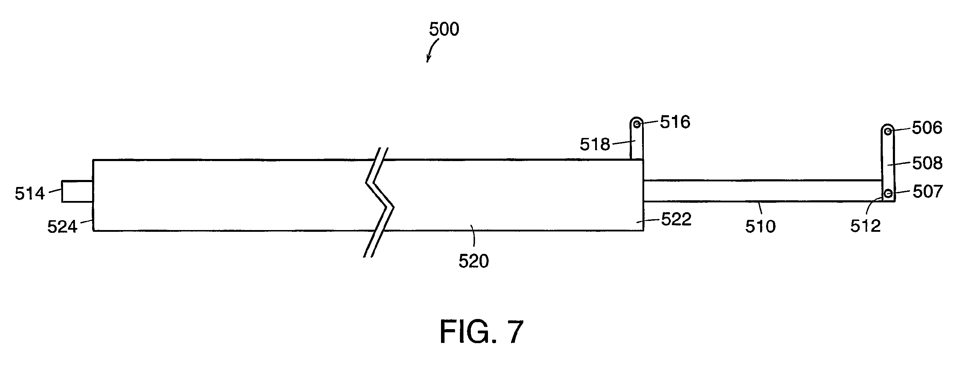

To wind, insert, position, and deploy the prostatic stent 100, a medical professional, such as, for example, a physician uses a delivery system 500. The delivery system 500 includes a rotatable element 510 and a stationary element 520, as shown in FIG. 7.

In the disclosed embodiment, the rotatable element 510 has an outer diameter less than the smaller diameter, d, of the middle portion 120 of the prostatic stent 100, so as to be insertable into the lumen 108 of the prostatic stent 100. The rotatable element 510 includes a first end 512 having a connection arm 508 that extends radially outward from the first end 512, and a second end 514 that is accessible to the medical professional for positioning the prostatic stent 100 within the urinary system of the patient. The connection arm 508 may pivot about a hinge 507, which attaches the connection arm 508 to the rotatable element 510. The connection arm 508 includes an opening 506 that permits connection between the proximal portion 110 of the prostatic stent 100 and the rotatable element 510. The stationary element 520 of the delivery system 500 includes a first end 522 having a connection arm 518 and a second end 524. The connection arm 518 extends radially outward from the first end 522 and includes an opening 516, which permits connection between the distal portion 115 of the prostatic stent 100 and the stationary element 520. In the disclosed embodiment, the stationary element 520 has an outer diameter larger than the diameter of the middle portion 120 and includes a lumen sized to receive the rotatable element 510.

Alternatively, in another embodiment of a delivery system, the stationary element may have an outer diameter less than or equal to the diameter of the middle portion and no lumen such that the rotatable and stationary elements extend substantially parallel to each other when attached to the prostatic stent 100. In another embodiment, the stationary element may have an outer diameter less than the smaller diameter, d, of the middle portion of the prostatic stent 100. In this embodiment, the stationary element is attachable to the proximal portion 110 of the prostatic stent 100 and the rotatable element, including a larger outer diameter and a lumen sized to receive the stationary element, connects to the distal portion 115 of the prostatic stent 100. In yet another embodiment, the stationary element may be replaced with another rotatable element. In this embodiment, the two rotatable elements rotate in opposing directions.

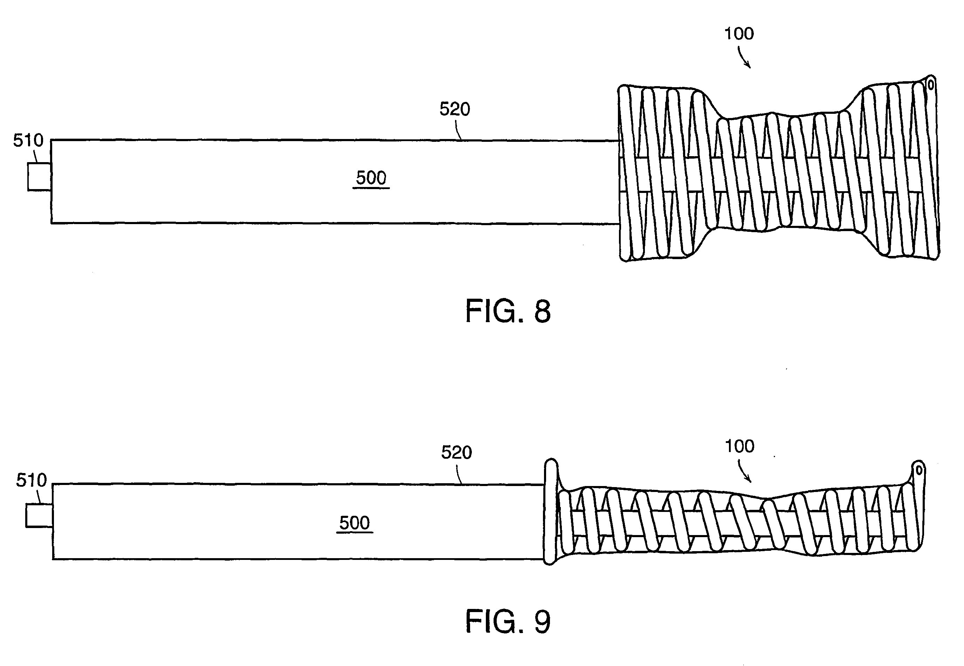

Before inserting the prostatic stent 100, either a manufacturer or a medical professional (typically, the manufacturer) uses the stationary and rotatable elements to reduce the outer diameter or width of the prostatic stent 100 such that injury or bruising to the urethra is substantially prevent during insertion and/or advancement of the prostatic stent 100 through the patient's urinary system. To connect the prostatic stent 100 with the delivery system 500 shown in FIG. 7, the manufacturer or medical professional attaches the proximal portion 110 of the prostatic stent 100 to the first end 512 via the connection arm 508 of the rotatable element 510 and the distal portion 115 to the first end 522 via the connection arm 518 of the stationary element 520, as shown in FIG. 8. In the disclosed embodiment, connection arms 508, 518 have openings 506, 516 to permit connection to hooks 106, 107 extending from the proximal and distal portions 110, 115 of the prostatic stent 100. Alternatively, the connection arms 508, 518 may be bent into hooks to permit connection with hooks 106, 107. Next, the manufacturer or the medical professional temporarily reduces the width of the prostatic stent 100 by twisting or ratcheting the rotatable element 520 that is attached to the proximal portion 110 of the prostatic stent 100 while simultaneously holding the stationary element 520 that is connected to the distal portion 115 of the prostatic stent 100 still. By twisting the rotatable element 510 in a first direction as shown in FIG. 9, the width of the prostatic stent 100 has decreased sufficiently to pass through the patient's urethra without significantly irritating or bruising the walls of the patient's urethra. As the width of the coil segment is reduced the length of the coil segment consequently extends as shown in FIGS. 5A, 5B, 8 and 9. Upon being released from the delivery system, the coil segment 105 expands and the length contracts until the prostatic stent 100 has returned to substantially its original dimensions.

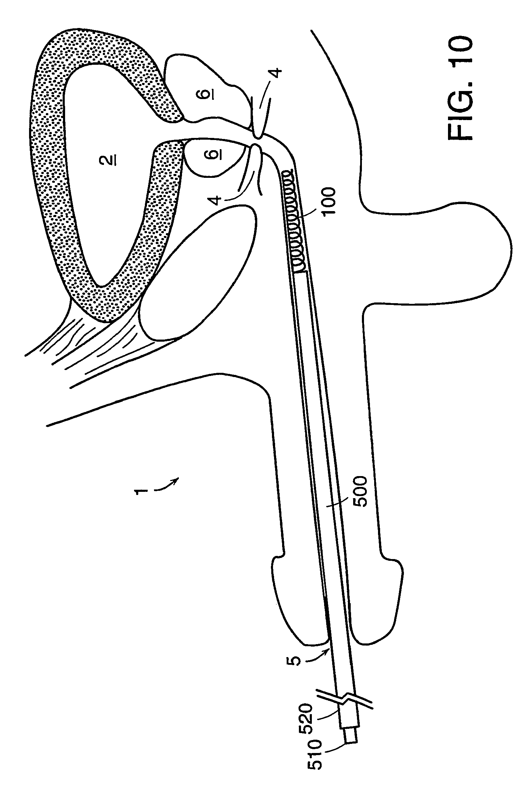

With the width of the prostatic stent 100 temporarily reduced, a medical professional inserts the prostatic stent 100 with attached delivery system 500 into the meatus of the patient, as shown in FIG. 10. To further protect the patient's urethra from irritation, the medical professional may insert a sheath into the patient's urethra prior to inserting the prostatic stent 100 attached to the delivery system 500. The sheath is a smooth tubular member sized to receive the prostatic stent 100 and the delivery system 500. Alternatively, the prostatic stent 100 attached to the delivery system 500 may be inserted into the sheath prior to the medical professional inserting the sheath including the prostatic stent 100 and delivery system 500 into the patient's body.

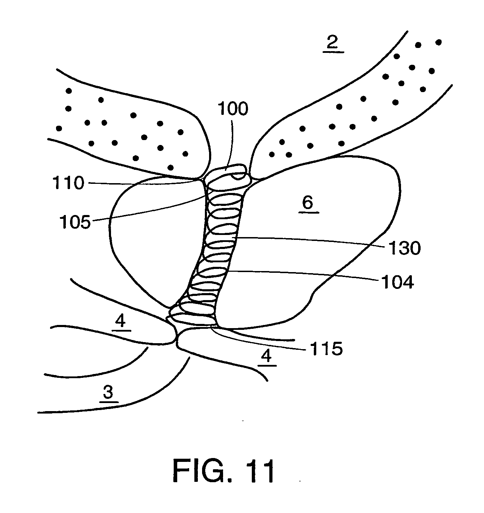

Referring to FIG. 11, the medical professional advances the prostatic stent 100 and the delivery system 500 through the patient's urinary system until the prostatic stent 100 is located substantially within the prostatic urethra with the proximal portion 110 located near the opening of the patient's bladder 2 and the distal portion 115 terminating prior to the proximal side of the patient's external sphincter 4 so as not to interfere with the normal operation of the external sphincter 4. After confirming proper placement of the prostatic stent 100 using radiographic techniques, the medical professional returns the prostatic stent 100 to its original or first width by rotating the rotatable element 510 in a second direction while restraining the stationary element 520 from moving. Once the prostatic stent 100 is returned to its original width, the proximal and distal portions 110, 115 with the larger diameter D, anchor the prostatic stent 100 within the proper position within the patient's urinary system. The medical professional is then able to detach the rotatable element 510 from the proximal portion 110 and the stationary element 520 from the distal portion 115 of the prostatic stent 100. With connection arm 508 no longer attached to the prostatic stent 100, the connection arm 508 is able to pivot about hinge 507, thereby allowing the medical professional to remove the rotatable element 510 and the connection arm 508 from the prostatic stent 100 without dislodging the positioning of the prostatic stent 100 after placement. The detached rotatable element 510 and stationary element 520 are then removed from the patient's urinary system.

The positioned prostatic stent 100 props open the patient's prostatic urethra, while simultaneously allowing the patient to control the opening and closing of his external sphincter 4. The positioned prostatic stent 100 has no parts or elements that communicate external to the patient's body during use, thereby reducing the high risk of infection associated with catheters. The radial strength provided by the coil segment 105 prevents the patient's prostatic urethra from collapsing due to the pressure created by the patient's enlarged prostate 6. The prostatic stent 100 is anchored in position by the proximal and distal portions 110,115 that inhibit migration of the prostatic stent 100. While positioned within the patient's urinary system 1, the imperforate webbing 130 prevents ingrowth of urethral tissue into the prostatic stent 100, thereby avoiding incorporation of the prostatic stent 100 into the patient's urethral walls and/or occlusion of the prostatic stent 100.

At some later time, the medical professional can remove the prostatic stent 100 by first inserting the delivery system 500 into the patient's urinary system and attaching the rotatable element 510 to the proximal portion 110 and the stationary element 520 to the distal portion 520 of the prostatic stent 100 in vivo. After the prostatic stent 100 is attached to the delivery system 500, the medical professional can decrease the width of the prostatic stent 100 by rotating the rotatable element 510 in the first direction as described above. With the delivery system 500 attached and the width of the prostatic stent 100 decreased, the medical professional removes the prostatic stent 100 from the patient's urethra. Alternatively, the medical professional may also use endoscopic means for removing the prostatic stent 100 from the patient's body.

Variations, modifications, and other implementations of what is described herein will occur to those of ordinary skill in the art without departing from the scope of the invention. Accordingly, the invention is not to be defined only by the preceding illustrative description.

## Claims

1. A stent (100, 200, 300) for use within a body lumen of a patient, comprising:
(a) a coil segment (105, 205, 305) defining a lumen (108) therethrough and including a distal portion (115), a middle portion (120), and a proximal portion (110), the coil segment (105, 205, 305) comprising a wound element including one or more windings (104, 204, 304) spaced from each other along at least a portion of the length of the coil segment (105, 205, 305) and being reducible in width at least to an extent needed to pass the stent (100, 200, 300) into the body lumen of the patient by winding the wound element, each of the distal (115) and proximal (110) portions including a diameter greater than a diameter of the middle portion (120) when the stent (100, 200, 300) is positioned and left within the body lumen of the patient; and
(b) a flexible polymer material encapsulating at least a portion of the coil segment and disposed between the spaced windings of the wound element to form an imperforate flexible webbing (130) between the windings (104, 204, 304) that inhibits ingrowth of body tissue between the windings (104, 204, 304) when the stent (100, 200, 300) is placed within the body lumen of the patient while also maintaining the lumen (108) of the coil segment (105, 205, 305) open.

2. The stent (100, 200. 300) of claim 1 wherein the wound element comprises a wire of a biocompatible material.

3. The stent (100, 200, 300) according to claim 2 wherein the biocompatible material is selected from the group consisting of stainless steel, titanium, a nickel-titanium alloy, or a polymer.

4. The stent (100, 200, 300) of claim 2 wherein a cross-sectional area of the wire is in the range of from about 7.9 x 10⁻³ millimeters² to about 7.1 millimeters².

5. The stent (100, 200, 300) of claim 1 wherein the spaced windings (104, 204, 304) are separated by a distance in the range of from about 0.5 millimeters to about 10 millimeters.

6. The stent (100, 200. 300) of claim 1 wherein each of the distal (115) and proximal (110) portions includes one or more hooks (106, 107) to permit connection to a delivery system (500).

7. The stent (100, 200, 300) of claim 1 wherein the flexible polymer material comprises a low durometer silicone.

8. The stent (100. 200, 300) of claim 7 wherein the low durometer silicone has a Shore A hardness in the range of from about 0 durometers to about 60 durometers.

9. A method of attaching a stent (100. 200, 300) to a delivery system (500), comprising:
(a) providing a stent (100, 200, 300) for use within a body lumen of a patient, comprising:
a coil segment (105, 205, 305) defining a lumen (108) therethrough and including a distal portion (115), a middle portion (120), and a proximal portion (110), the coil segment (105, 205, 305) comprising a wound element including one or more windings (104, 204, 304) spaced from each other along at least a portion of the length of the coil segment (105, 205, 305) and being reducible in width at least to an extent needed to pass the stent (100, 200, 300) into the body lumen of the patient by winding of the wound element, each of the distal (115) and proximal (110) portions including a diameter greater than a diameter of the middle portion (120) when the stent (100, 200, 300) is positioned and left within the body lumen of the patient, and
a flexible polymer material encapsulating at least a portion of the coil segment and disposed between the spaced windings of the wound element to form an imperforate flexible webbing (130) between the windings (104, 204, 304) that inhibits ingrowth of body tissue between the spaced windings (104, 204, 304) when the stent (100, 200, 300) is placed within the body lumen of the patient while also maintaining the lumen (108) of the coil segment (105, 205, 305) open;
(b) providing a delivery system (500) comprising:
a first element (510) having an outer diameter smaller than the diameter of the middle portion (120) of the stent (100, 200, 300) and including a first end (512), a second end (514), and a connection member (508) extending out from the first end (512), and
a second element (520) including a first end (522), a second end (524), and a connection member (518) extending out from the first end (522), at least one of the first (510) and second (520) elements of the delivery system (500) being rotatable;
(c) placing the first element (510) of the delivery system (500) within the lumen (108) of the coil segment (105, 205, 305);
(d) attaching the connection member (508) of the first element (510) to the proximal portion (110) of the stent (100, 200, 300);
(e) attaching the connection member (518) of the second element (520) to the distal portion (115) of the stent (100, 200, 300); and
(f) rotating at least one of the first (510) and the second (520) elements to further wind the wound element to reduce the width of the stent (100, 200, 300) at least to an extent needed to pass the stent (100. 200, 300) into the urethra of the patient.

10. The method of claim 9 wherein the connection member (508) of the first element (510) comprises an arm extending radially outward from the first end (512) and includes an opening (506) sized to receive a hook (106) extending from the proximal portion (110) of the stent (100, 200, 300).

11. The method of claim 9 wherein the connection member (518) of the second element (520) comprises an arm extending radially outward from the first end (522) and includes an opening (516) sized to receive a hook (107) extending from the distal portion (115) of the stent (100, 200, 300).

12. The method of claim 9 wherein the second element (520) defines a lumen extending therethrough and sized to receive the first element (510).

## Patentansprüche

1. Ein Stent (100, 200, 300) zur Verwendung in einem Körperhohlraum eines Patienten, aufweisend:
(a) Ein Spiralsegment (105, 205, 305), welches einen durchgehenden Hohlraum (108) definiert und einen distalen Abschnitt (115), einen mittleren Abschnitt (120) und einen proximalen Abschnitt (110) aufweist, wobei das Spiralsegment (105, 205, 305) ein gewundenes Element mit einer oder mehreren Windungen (104, 204, 304) aufweist, die voneinander entlang wenigstens eines Abschnitts der Länge des Spiralsegments (105, 205, 305) beabstandet sind und die in der Breite zumindest in einem solchen Ausmaß reduzierbar sind, welches erforderlich ist, um den Stent (100, 200, 300) in den Körperhohlraum des Patienten durch aufwickeln des gewundenen Elements einzubringen, wobei jeder der distalen (115) und proximalen (110) Abschnitte einen größeren Durchmesser als der Durchmesser des mittleren Abschnitts (120) aufweist, wenn der Stent (100, 200, 300) innerhalb des Körperhohlraums des Patienten angeordnet und zurückgelassen wird; und
(b) ein flexibles Polymermaterial, welches wenigstens einen Abschnitt des Spiralsegments verkapselt und zwischen den beabstandeten Windungen des gewundenen Elements zum Bilden einer unperforierten flexiblen Haut (130) zwischen den Windungen (104, 204, 304) angeordnet ist, welche das Einwachsen von Körpergewebe zwischen den Windungen (104, 204, 304) verhindert, wenn der Stent (100, 200, 300) innerhalb des Körperhohlraums des Patienten platziert ist, während außerdem der Hohlraum (108) des Spiralsegments (105, 205, 305) offen gehalten wird.

2. Der Stent (100, 200, 300) gemäß Anspruch 1, wobei das gewundene Element einen Draht eines biokompatiblen Materials aufweist.

3. Der Stent (100, 200, 300) gemäß Anspruch 2, wobei das biokompatible Material ausgewählt ist aus der Gruppe von rostfreiem Stahl, Titan, einer Nickel-Titan-Legierung oder eines Polymers.

4. Der Stent (100, 200, 300) gemäß Anspruch 2, wobei die Querschnittsfläche des Drahtes in dem Bereich von etwa 7,9 x 10⁻³ mm² bis etwa 7,1 mm² liegt.

5. Der Stent (100, 200, 300) gemäß Anspruch 1, wobei die beabstandeten Windungen (104, 204, 304) durch einen Abstand in dem Bereich von 0,5 mm bis etwa 10 mm getrennt sind.

6. Der Stent (100, 200, 300) gemäß Anspruch 1, wobei die distalen (115) und proximalen (110) Abschnitte jeweils einen oder mehrere Haken (106, 107) aufweisen, die eine Verbindung zu einem Einbringungssystem (500) erlauben.

7. Der Stent (100, 200, 300) gemäß Anspruch 1, wobei das flexible Polymermaterial ein Silikon geringer Härte aufweist.

8. Der Stent (100, 200, 300) gemäß Anspruch 7, wobei das Silikon geringer Härte eine Shore A Härte in dem Bereich von etwa 0 Durometer-Härten bis etwa 60 Durometer-Härten aufweist.

9. Ein Verfahren zum Anbringen eines Stents (100, 200, 300) an einem Einbringungssystem (500) aufweisend:
(a) Bereitstellen eines Stent (100, 200, 300) zur Verwendung in einem Körperhohlraum eines Patienten, aufweisend:
Ein Spiralsegment (105, 205, 305) welches einen durchgehenden Hohlraum (108) definiert und einen distalen Abschnitt (115), einen mittleren Abschnitt (120) und einen proximalen Abschnitt (110) aufweist, wobei das Spiralsegment (105, 205, 305) ein gewundenes Element mit einer oder mehreren Windungen (104, 204, 304) aufweist, die voneinander entlang wenigstens eines Abschnitts der Länge des Spiralsegments (105, 205, 305) beabstandet sind und die in ihrer Breite zumindest in einem solchen Maß reduzierbar sind, um den Stent (100, 200, 300) in den Körperhohlraum des Patienten einzubringen, indem die gewundenen Elemente aufgewickelt werden, wobei jeder der distalen (115) und proximalen (110) Abschnitte einen größeren Durchmesser aufweist als der Durchmesser des mittleren Abschnitts (120), wenn der Stent (100, 200, 300) in dem Körperhohlraum des Patienten angeordnet und dort zurückgelassen ist, und
ein flexibles Polymermaterial, welches wenigstens einen Abschnitt des Spiralsegments verkapselt und zwischen den beabstandeten Windungen des gewundenen Elements zum Bilden einer unperforierten flexiblen Haut (130) zwischen den Windungen (104, 204, 304) angeordnet ist, um das Einwachsen von Körpergewebe zwischen den beabstandeten Windungen (104, 204, 304) zu verhindern, wenn der Stent (100, 200, 300) innerhalb des Körperhohlraums des Patienten platziert ist, während außerdem der Hohlraum (108) des Spiralsegments (105, 205, 305) offen gehalten wird;
(b) Bereitstellen eines Einbringungssystems (500), aufweisend:
ein erstes Element (510) mit einem Außendurchmesser, der kleiner als der Durchmesser des mittleren Abschnitts (120) des Stents (100, 200, 300) ist und ein erstes Ende (512), ein zweites Ende (514) und ein Verbindungsteil (508) aufweist, welches sich aus dem ersten Ende (512) erstreckt und
ein zweites Element ((520) mit einem ersten Ende (522), einem zweiten Ende (524) und einem Verbindungsteil (518), welches sich aus dem ersten Ende (522) erstreckt, wobei wenigstens eines der ersten (510) und zweiten (520) Elemente des Einbringungssystem (500) drehbar ist;
(c) Platzieren des ersten Elements (510) des Einbringungssystems (500) innerhalb des Hohlraums (108) des Spiralsegments (105, 205, 305);
(d) Anbringen des Verbindungsteils (508) des ersten Elements (510) an dem proximalen Abschnitt (110) des Stents (100, 200, 300) ;
(e) Anbringen des Verbindungsteils (518) des zweiten Elements (520) an dem distalen Abschnitt (115) des Stents (100, 200, 300); und
(f) Drehen von wenigstens einem des ersten (510) und zweiten (520) Elements, um das gewundene Element zur Verringerung der Breite des Stents (100, 200, 300) in einem solchen Maß weiter aufzuwickeln, wie es zum Einbringen des Stents (100, 200, 300) in die Urethra des Patienten erforderlich ist.

10. Das Verfahren gemäß Anspruch 9, wobei das Verbindungsteil (508) des ersten Elements (510) einen Arm aufweist, der sich radial nach außen von dem ersten Ende (512) erstreckt und eine Öffnung (506) aufweist, die in Ihrer Grösse zur Aufnahme eines Hakens (106) bemessen ist, der sich vom dem proximalen Abschnitt (110) des Stents (100, 200, 300) erstreckt.

11. Das Verfahren gemäß Anspruch 9, wobei das Verbindungsteil (518) des zweiten Elements (520) einen Arm aufweist, der sich radial nach außen vom dem ersten Ende (522) erstreckt und eine Öffnung (516) aufweist, die in ihrer Größe zur Aufnahme eines Hakens (107) ausgebildet ist, welcher sich von dem distalen Abschnitt (115) des Stents (110, 200, 300) erstreckt.

12. Das Verfahren gemäß Anspruch 9, wobei das zweite Element (520) einen durchgehenden Hohlraum definiert und bemessen ist, um das erste Element (510) aufzunehmen.

## Revendications

1. Stent (100, 200, 300) pour usage dans une lumière du corps d'un patient, comprenant :
(a) un segment spiralé (105, 205, 305) définissant une lumière traversante (108) et comprenant une partie distale (115), une partie centrale (120) et une partie proximale (110), le segment spiralé (105, 205, 305) comprenant un élément enroulé comportant un ou plusieurs enroulements (104, 204, 304) espacés l'un de l'autre sur au moins une partie de la longueur du segment spiralé (105, 205, 305) et pouvant être réduit en largeur au moins dans une certaine mesure nécessaire pour faire passer le stent (100, 200, 300) dans la lumière du corps du patient en enroulant l'élément enroulé, chacune des parties distale (115) et proximale (110) comprenant un diamètre plus grand que le diamètre de la partie centrale (120) lorsque le stent (100, 200, 300) est positionné et laissé dans la lumière du corps du patient; et
(b) un matériau polymère flexible encapsulant au moins une partie du segment spiralé et disposé entre les enroulements espacés de l'élément enroulé pour former une nappe flexible non perforée (130) entre les enroulements (104, 204, 304) qui inhibe la croissance interne du tissu corporel entre les enroulements (104, 204, 304) lorsque le stent (100, 200, 300) est placé dans la lumière du corps du patient tout en maintenant également la lumière (108) du segment spiralé (105, 205, 305) ouverte.

2. Stent (100, 200, 300) selon la revendication 1, dans lequel l'élément enroulé comprend un fil d'un matériau biocompatible.

3. Stent (100, 200, 300) selon la revendication 2, dans lequel le matériau biocompatible est choisi dans le groupe constitué de l'acier inoxydable, du titane, d'un alliage de nickel-titane ou d'un polymère.

4. Stent (100, 200, 300) selon la revendication 2, dans lequel une surface en coupe transversale du fil se situe dans la plage d'environ 7,9 x 10⁻³ mm² à environ 7,1 mm².

5. Stent (100, 200, 300) selon la revendication 1, dans lequel les enroulements espacés (104, 204, 304) sont séparés d'une distance dans la plage d'environ 0,5 mm à environ 10 mm.

6. Stent (100, 200, 300) selon la revendication 1, dans lequel chacune des parties distale (115) et proximale (110) comprend un ou plusieurs crochets (106, 107) pour permettre un raccordement avec un système de délivrance (500).

7. Stent (100, 200, 300) selon la revendication 1, dans lequel le matériau polymère flexible comprend une silicone de faible dureté au duromètre.

8. Stent (100, 200, 300) selon la revendication 7, dans lequel la silicone de faible dureté au duromètre a une dureté Shore A dans la plage d'environ 0 à environ 60 au duromètre.

9. Procédé de fixation d'un stent (100, 200, 300) à un système de délivrance (500), comprenant :
(a) la mise en oeuvre d'un stent (100, 200, 300) pour usage dans une lumière du corps d'un patient, comprenant :
un segment spiralé (105, 205, 305) définissant une lumière traversante (108) et comprenant une partie distale (115), une partie centrale (120) et une partie proximale (110), le segment spiralé (105, 205, 305) comprenant un élément enroulé comportant un ou plusieurs enroulements (104, 204, 304) espacés l'un de l'autre sur au moins une partie de la longueur du segment spiralé (105, 205, 305) et pouvant être réduit en largeur au moins dans une certaine mesure nécessaire pour faire passer le stent (100, 200, 300) dans la lumière du corps du patient en enroulant l'élément enroulé, chacune des parties distale (115) et proximale (110) comprenant un diamètre plus grand que le diamètre de la partie centrale (120) lorsque le stent (100, 200, 300) est positionné et laissé dans la lumière du corps du patient ; et
un matériau polymère flexible encapsulant au moins une partie du segment spiralé et disposé entre les enroulements espacés de l'élément enroulé pour former une nappe flexible non perforée (130) entre les enroulements (104, 204, 304) qui inhibe la croissance interne du tissu corporel entre les enroulements (104, 204, 304) lorsque le stent (100, 200, 300) est placé dans la lumière du corps du patient tout en maintenant également la lumière (108) du segment spiralé (105, 205, 305) ouverte ;
(b) la mise en oeuvre d'un système de délivrance (500) comprenant :
un premier élément (510) ayant un diamètre externe inférieur au diamètre de la partie centrale (120) du stent (100, 200, 300) et comprenant une première extrémité (512), une seconde extrémité (514) et un élément de raccordement (508) s'étendant hors de la première extrémité (512), et
un second élément (520) comprenant une première extrémité (522), une seconde extrémité (524) et un élément de raccordement (518) s'étendant hors de la première extrémité (522), au moins l'un des premier (510) et second (520) éléments du système de délivrance (500) étant rotatif;
(c) le placement du premier élément (510) du système de délivrance (500) dans la lumière (108) du segment spiralé (105, 205, 305) ;
(d) la fixation de l'élément de raccordement (508) du premier élément (510) sur la partie proximale (110) du stent (100, 200, 300) ;
(e) la fixation de l'élément de raccordement (518) du second élément (520) sur la partie distale (115) du stent (100, 200, 300) ; et
(f) la rotation d'au moins l'un des premier (510) et second (520) éléments pour enrouler davantage l'élément enroulé afin de réduire la largeur du stent (100, 200, 300) au moins dans une certaine mesure nécessaire pour faire passer le stent (100, 200, 300) dans l'urètre du patient.

10. Procédé selon la revendication 9, dans lequel l'élément de raccordement (508) du premier élément (510) comprend un bras s'étendant radialement vers l'extérieur de la première extrémité (512) et comprend une ouverture (506) dimensionnée pour recevoir un crochet (106) s'étendant de la partie proximale (110) du stent (100, 200, 300).

11. Procédé selon la revendication 9, dans lequel l'élément de raccordement (518) du second élément (520) comprend un bras s'étendant radialement vers l'extérieur de la première extrémité (522) et comprend une ouverture (516) dimensionnée pour recevoir un crochet (107) s'étendant de la partie distale (115) du stent (100, 200, 300).

12. Procédé selon la revendication 9, dans lequel le second élément (520) définit une lumière qui le traverse et dimensionnée pour recevoir le premier élément (510).
